(19) Europäisches Patentamt
European Patent Office
Office européen des brevets

(11) **EP 3 271 449 B1**

(12) **FASCICULE DE BREVET EUROPEEN**

(45) Date de publication et mention
de la délivrance du brevet:
**04.05.2022 Bulletin 2022/18**

(21) Numéro de dépôt: **16712394.2**

(22) Date de dépôt: **18.03.2016**

(51) Classification Internationale des Brevets (IPC):
***C12M 1/00*** *(2006.01)*

(52) Classification Coopérative des Brevets (CPC):
**C12M 21/02; C12M 23/22; C12M 31/02;
C12M 41/06**

(86) Numéro de dépôt international:
**PCT/FR2016/050603**

(87) Numéro de publication internationale:
**WO 2016/151219 (29.09.2016 Gazette 2016/39)**

(54) **BIOREACTEUR POUR MICROALGUES**

BIOREAKTOR FÜR MIKROALGEN

BIOREACTOR FOR MICROALGAE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.03.2015 FR 1552343**

(43) Date de publication de la demande:
**24.01.2018 Bulletin 2018/04**

(73) Titulaires:
• **INRIA - Institut National de Recherche en
Informatique et en Automatique**
**78150 Le Chesnay (FR)**
• **Centre National de la Recherche Scientifique
(CNRS)**
**75016 Paris (FR)**
• **Sorbonne Université**
**75006 Paris (FR)**

(72) Inventeurs:
• **MAIRET, Francis**
**06560 Valbonne (FR)**
• **BERNARD, Olivier**
**06510 Carros (FR)**
• **SCIANDRA, Antoine**
**06230 Villefranche sur Mer (FR)**
• **PRUVOST, Eric**
**06100 Nice (FR)**
• **COMBE, Charlotte**
**06700 Saint Laurent du Var (FR)**

(74) Mandataire: **Cabinet Netter**
**36, avenue Hoche**
**75008 Paris (FR)**

(56) Documents cités:
**WO-A1-2010/085853    WO-A2-2008/033573
WO-A2-2011/080345    US-A1- 2012 171 733**

• **BOJAN TAMBURIC ET AL: "Action spectra of
oxygen production and chlorophyll a
fluorescence in the green microalga
Nannochloropsis oculata", BIORESOURCE
TECHNOLOGY., vol. 169, 8 juillet 2014
(2014-07-08), pages 320-327, XP055249720, GB
ISSN: 0960-8524, DOI:
10.1016/j.biortech.2014.07.008**

**Description**

La présente invention concerne un bioréacteur pour produire de la biomasse de microalgues dans un milieu de culture. L'invention se rapporte également à un procédé de production de biomasse de microalgues.

Il existe des micro-organismes unicellulaires phototrophes procaryotes et eucaryotes, regroupés communément sous le terme de microalgues. Les micro-organismes photosynthétiques procaryotes sont représentés par les cyanobactéries (parfois appelées « algues bleu-vert »). Les micro-organismes photosynthétiques eucaryotes sont diversifiés et sont représentés par une multitude de classes parmi lesquelles on peut citer les chlorophycées, les diatomées, les chrysophycées, les coccolithophycées, les euglénophycées et les rhodopycées. D'une manière générale, la taille d'une cellule de microalgue est comprise entre 1 $\mu$m et 100 $\mu$m.

On estime aujourd'hui qu'il existe plus d'un million d'espèces de microalgues dont quelques dizaines de milliers d'espèces sont référencées. Les microalgues sont ubiquistes et on les trouve aussi bien dans les eaux douces et que dans les eaux saumâtres et marines.

La production de microalgues est un secteur qui prend de plus en plus d'ampleur. En effet, les microalgues synthétisent de nombreux produits de natures différentes parmi lesquels on peut citer les protéines, les antioxydants, les pigments, les acides gras polyinsaturés à longues chaînes DHA (acide docosahexaénoïque) et EPA (acide eicosapentaénoïque).

Ainsi, les microalgues trouvent une application dans plusieurs domaines technologiques et notamment dans l'industrie cosmétique, l'industrie pharmaceutique, l'aquaculture, l'industrie des alicaments ou des compléments alimentaires.

Par ailleurs, les microalgues sont utilisées dans la production de bioénergie. En effet, les microalgues ont une capacité à capter l'énergie lumineuse pour fixer et métaboliser le carbone inorganique à partir du dioxyde de carbone ($CO_2$) dans des molécules énergétiques. Les microalgues présentent donc des capacités épuratoires importantes. De plus, le couplage des microalgues avec le $CO_2$ et le fait que les microalgues sont souvent riches en sucres ou en huiles ont pour conséquence que les microalgues présentent un grand intérêt dans la production de biocarburants.

Les microalgues peuvent être cultivées en utilisant de la lumière naturelle (lumière solaire) ou de la lumière artificielle. Il existe des systèmes de culture ouverts de type bassin de culture (aussi appelé bassin « raceway ») et des systèmes de culture fermés de type photobioréacteur. Dans les systèmes de culture se trouve généralement une cuve emplie d'un milieu de culture. Les microalgues sont dispersées dans le milieu de culture. WO 2010/085853 divulgue un photobioréacteur avec un matériel luminescent qui a pour but de convertir une source lumineuse naturelle peu absorbée à une source lumineuse de différente longueurs d'ondes mieux absorbées par les microalgues, ce qui permet un meilleur rendement.

Pour une bonne production de microalgues en condition phototrophe et indépendamment du système de culture, il faut fournir des nutriments (azote, phosphore, souffre, oligo-éléments, vitamines) et de la lumière aux microalgues. En effet, en présence des nutriments nécessaires, les microalgues peuvent convertir l'énergie lumineuse en métabolisant le $CO_2$ et ainsi produire de l'oxygène et de la biomasse algale (matière organique de microalgues). Il s'agit de la photosynthèse.

Pour obtenir une bonne productivité, il est donc important d'éclairer les microalgues. D'une manière générale, une lumière à longueur d'onde fortement absorbée par les microalgues est nécessaire pour obtenir un taux de croissance élevé. En pratique il s'agit de lumières contenant des longueurs d'onde dans le bleu et dans le rouge.

Les publications Light requirements in microalgal photobioreactors: an overview of biophotonic aspects - Carvalho et al., Appl Microbiology and Biotechnology, 2011, vol. 89, no. 5: 1275-1288 et Light emitting diodes (LEDs) applied to microalgal production - Schulze et al., Trends in Biotechnology, 2014, vol. 32, no. 8: 422-430 décrivent l'utilisation de la lumière dans les systèmes de culture des microalgues.

Dans un bioréacteur, les cellules de microalgues proches de la source lumineuse (par exemple en surface ou à la périphérie de la cuve) reçoivent un excès de photons. L'excès de photons absorbés par les microalgues entraîne une baisse de l'efficacité photosynthétique. Il s'agit d'une dissipation de l'énergie appelée dissipation non-photochimique (en anglais : non-photochemical quenching). Le phénomène de la dissipation non-photochimique est notamment décrit dans la publication Non-Photochemical Quenching. A Response to Excess Light Energy - Müller et al., Plant Physiol, 2001, vol. 125, no. 4: 1558-1566. Un excès d'absorption de photons peut également provoquer une dégradation de l'appareil photosynthétique, phénomène appelé photoinhibition.

Dans les cultures à haute densité, c'est-à-dire à forte concentration de cellules, la lumière incidente est majoritairement absorbée par les cellules proches de la source lumineuse. En d'autres termes, une grande partie de la lumière incidente ne parvient pas à pénétrer en profondeur dans la cuve. Il se crée en conséquence un gradient de lumière au sein du milieu de culture.

Pour ces cultures à haute densité, une quantité importante de cellules se retrouve en profondeur de la cuve et donc à distance de la surface ou de la périphérie. Ces cellules en profondeur peuvent se retrouver dans l'obscurité du fait que la lumière ne pénètre pas en profondeur dans la cuve. La fixation du carbone par photosynthèse ne compense alors plus les pertes d'énergie par la respiration cellulaire, basée sur la dégradation de sucres qui ont été synthétisés lors de la photosynthèse. Il faut donc prévoir d'augmenter le mélange pour équilibrer la distribution de lumière vers les cellules ou placer des sources de lumière à l'intérieur de la cuve.

Pour résumer, les cultures à haute densité présentent un problème de surexposition lumineuse des cellules proches de la source lumineuse et un problème de sous-exposition des cellules situées en profondeur du milieu de culture.

C'est pourquoi les solutions de l'état de la technique proposent des systèmes de culture avec des sources de lumière à forte intensité lumineuse (préférablement réparties en périphérie et/ou à l'intérieur de la cuve) combinés à des outils de mélange performants. Ces systèmes sont coûteux en énergie.

Il existe par ailleurs une autre approche qui consiste à réduire la taille des antennes des microalgues. La réduction de taille d'antennes est réalisée par modification génétique des microalgues. Le but de cette approche est de rendre les microalgues plus transparentes. Chaque cellule capte ainsi une quantité moindre de lumière ce qui réduit le phénomène de dissipation non-photochimique. Les cellules localisées dans les zones les plus éloignées de la source lumineuse sont donc moins ombragées. Toutefois, cette approche nécessite des méthodes de génie génétique complexes et coûteuses.

La présente invention vient améliorer la situation.

À cet effet, l'invention vient introduire un bioréacteur comportant une source de lumière et une cuve comprenant un milieu de culture dans laquelle est dispersée une culture cellulaire constituée de cellules d'algues, ladite culture cellulaire d'algues présentant une concentration supérieure à 0,1 g/L dans le milieu de culture, chaque cellule d'algue présentant une absorption minimale dans une plage de longueurs d'onde spécifique de lumière, ladite source étant capable d'émettre une lumière incidente en direction de la cuve constituée d'au moins 60% de photons dont la longueur d'onde est comprise dans la plage de longueur d'onde spécifique de lumière.

D'autres avantages et caractéristiques de l'invention apparaîtront à la lecture de la description détaillée ci-après et sur les dessins annexés sur lesquels :

- la figure 1 montre un graphique de spectres d'absorption de différentes microalgues ;
- la figure 2 montre un schéma de production de biomasse de microalgues dans un système de culture de l'état de la technique ;
- la figure 3 montre un graphique de l'absorbance d'un milieu de culture comprenant des microalgues, en fonction de la longueur d'onde d'une lumière incidente ;
- la figure 4 montre un graphique de l'intensité d'une lumière incidente par rapport à l'intensité d'une lumière sortante en fonction de la longueur d'onde ;
- la figure 5 montre un graphique de gradient de lumière dans un milieu de culture de microalgues ;
- la figure 6 montre des simulations de l'absorbance et de quatre lumières incidentes différentes (ayant un nombre égal de photons) en fonction de la longueur d'onde ;
- la figure 7 montre la comparaison entre le taux de croissance spécifique et la production nette de biomasse d'une culture cellulaire d'algue en fonction de la concentration de biomasse pour différentes lumières incidentes ; et
- la figure 8 montre un schéma de production de biomasse de microalgues dans un bioréacteur selon l'invention.

Les dessins et la description ci-après contiennent, pour l'essentiel, des éléments de caractère certain. Ils font partie intégrante de la description, et pourront donc non seulement servir à mieux faire comprendre la présente invention, mais aussi contribuer à sa définition, le cas échéant.

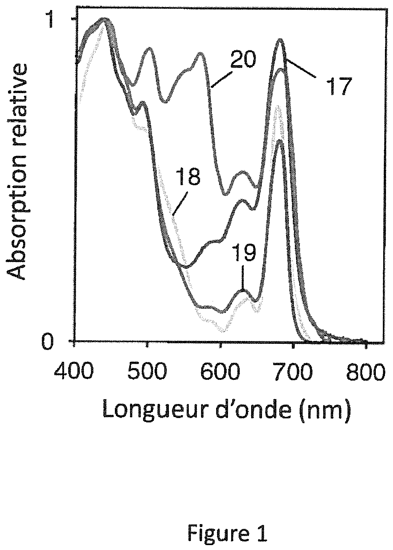

La figure 1 montre un graphique de spectres d'absorption de différentes microalgues. Ce graphique est tiré de la publication Colorful niches of phototrophic microorganisms shaped by vibrations of the water molecule - Stomp et al., The ISME journal, vol. 1, no 4 :271-282, 2007.

La courbe 17 montre le spectre d'absorption normalisé d'une algue verte *Chlamydomonas sp.* de la classe Chlorophyceae ; la courbe 18 montre le spectre d'absorption normalisé de *Phaeodactylum tricornutum* de la classe Bacillariophyceae (ou Diatomophyceae); la courbe 19 montre le spectre d'absorption normalisé d'une Chrysophyceae *Isochrysis sp.* de la classe Chrysophyceae ; et la courbe 20 montre le spectre d'absorption normalisé d'une algue rouge *Palmaria palmata* de la classe Florideophyceae.

La figure 1 montre que chaque type de microalgue présente une absorption minimale pour une lumière de longueur d'onde donnée. En effet, l'algue verte 17 *Chlamydomonas sp.* présente une absorption minimale comprise entre 500 nm et 600 nm, le point d'absorption minimale se situe à environ 550 nm ; l'algue de la classe diatomée 18 *Phaeodactylum tricornutum* présente une absorption minimale comprise entre 550 nm et 650 nm, le point d'absorption minimale se situe à environ 600 nm ; l'algue de la classe chrysophyte 19 *Isochrysis sp.* présente une absorption minimale comprise entre 550 nm et 650 nm, le point d'absorption minimale se situe à environ 600 nm ; l'algue rouge 20 *Palmaria palmata* présente une absorption minimale comprise entre 580 nm et 650 nm, le point d'absorption minimale se situe à environ 600 nm.

Ces microalgues présentent également des pics d'absorption maximale. Des premiers pics sont observés pour des lumières de longueur d'onde comprise entre 430 nm et 470 nm (lumière bleue) et des deuxièmes pics sont observés pour des lumières de longueur d'onde comprise entre 650 nm et 700 nm (lumière rouge).

Selon les enseignements de l'état de la technique, pour obtenir une production élevée de biomasse d'une culture de microalgues, une lumière incidente L à longueur d'onde fortement absorbée serait utilisée.

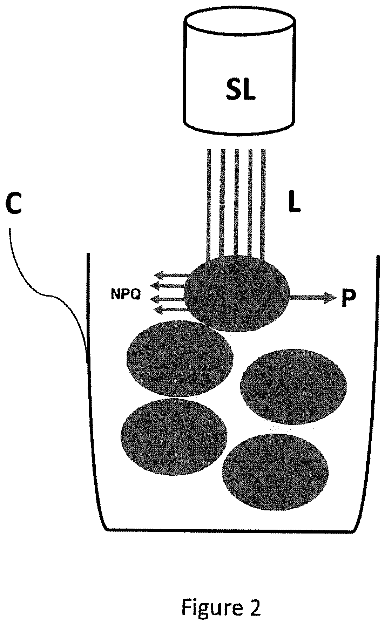

La figure 2 montre un schéma de production de biomasse de microalgues dans les systèmes de culture de l'état de la technique.

Une source de lumière SL émet une lumière incidente L. La lumière incidente L éclaire les cellules de microalgues situées à la périphérie d'une cuve C de bioréacteur. La lumière incidente L fournit des photons aux cellules de microalgues situées proche de la source lumineuse (en périphérie).

La lumière incidente est émise avec une longueur d'onde fortement absorbée par les cellules de microalgues. L'intensité de la lumière incidente a pour conséquence le phénomène de dissipation non-photochimique NPQ (en anglais : Non-Photochemical Quenching), décrit plus haut.

Les cellules de microalgues situées en profondeur de la cuve C ne sont pas éclairées et ne reçoivent donc pas de photons.

La production P de biomasse n'est pas satisfaisante.

Certains systèmes de culture de l'état de la technique utilisent un outil de mélange amélioré pour ainsi favoriser une circulation des microalgues au sein de la cuve. Ceci permet au moins partiellement aux cellules de microalgues initialement situées au sein de la cuve d'être exposées à la lumière incidente en surface ou en périphérie de la cuve. Le mélange de la culture cellulaire a pour conséquence une succession, à l'échelle d'une cellule de microalgue, de périodes éclairées et obscures.

Toutefois, le mélange efficace d'un milieu de culture représente un coût énergétique important.

De plus, dans les cultures cellulaires à haute densité, un mélange du milieu de culture ne permet généralement pas d'éviter complètement les effets délétères causés par le gradient de lumière (photoinhibition en surface ou périphérie et respiration importante en profondeur).

La présente invention propose une approche radicalement différente de celle de l'état de la technique. Plus encore, la présente invention met en œuvre le contraire des enseignements et solutions proposées dans l'état de la technique. En effet, l'invention utilise des lumières spectrales de longueurs d'ondes peu absorbées par les microalgues.

La Demanderesse a découvert non sans surprise que les problèmes liés à la mauvaise répartition des photons au sein d'un milieu de culture à haute densité (gradient de lumière) sont fortement réduits par l'utilisation d'une lumière peu absorbée par les microalgues. L'efficacité photosynthétique globale du système de culture est ainsi augmentée.

Alors que pour les cultures à faible densité ce type de lumière entraîne une croissance plus lente, due à un faible nombre de photons absorbés, pour les cultures à haute densité, ce type de lumière favorise la pénétration des photons dans le milieu de culture. Il s'ensuit que l'utilisation d'une lumière de longueur d'onde peu absorbée permet une meilleure répartition de l'énergie lumineuse. En conséquence, en surface, les pertes par dissipation et les effets de photoinhibition qui apparaissent lorsqu'une cellule reçoit trop de photons sont évités. Une lumière de longueur d'onde peu absorbée permet donc d'éclairer un volume plus important du milieu de culture que les lumières utilisées dans l'état de la technique.

Par ailleurs, le cycle cellulaire des microalgues est régulé entre autre par des photorécepteurs sensibles à la lumière bleu et rouge (cf. Photoreceptors in algae, Rudiger, W and Lopez-Figueroa, F, Photochemistry and photobiology, vol. 55, pp. 949-954, 1992). L'absence de ces longueurs d'ondes dans l'éclairement a pour effet de retarder le cycle cellulaire (sans toutefois influencer la photosynthèse), entraînant des cellules plus grosses, présentant un contenu plus important en carbone de réserve.

L'invention permet donc d'améliorer la productivité du système, de réduire l'énergie lumineuse nécessaire et les coûts associés, tout en réduisant l'énergie nécessaire à la récolte de la biomasse de microalgues.

Ce qui a motivé la Demanderesse est que le rendement quantique, c'est-à-dire le nombre de molécules d'oxygène synthétisées par photon absorbé, est peu sensible au type de lumière incidente. Le rendement quantique est donc quasi indépendant de la longueur d'onde, ceci est notamment décrit dans la publication scientifique Action spectra of oxygen production and chlorophyll a fluorescence in the green microalga Nannochloropsis oculata - Tamburic et al., Bioresource Technology vol. 169 :320-327, 2014.

Ainsi, en termes de métabolisation, les photons absorbés issus d'une lumière à longueur d'onde peu absorbée ont la même efficacité que les photons absorbés issus d'une lumière à longueur d'onde fortement absorbée. Ceci est notamment décrit dans la publication scientifique Aquatic photosynthesis, Falkowski, P. G., & Raven, J. A. , Princeton University Press, (2013).

Par exemple, les photons verts qui sont généralement peu absorbés par les microalgues chlorophytes, ont le même effet (une fois captés par les cellules) que les photons rouges qui sont généralement fortement absorbés par les microalgues. En bref, une fois qu'un photon est capté par une microalgue, il sera également utilisé pour le métabolisme, indépendamment de sa longueur d'onde.

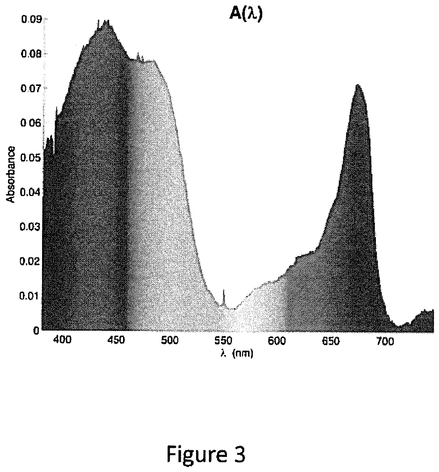

La figure 3 illustre l'absorbance d'une culture cellulaire de la Chlorophyceae *Dunalliela salina* en fonction de la longueur d'onde d'une lumière incidente. Il ressort de la figure 3 que ce type d'algue verte présente généralement peu d'absorbance pour des longueurs d'ondes comprises entre 550 nm et 600 nm.

Les phénomènes d'optique, d'absorbance et de métabolisation de photons dans une cuve de bioréacteur sont détaillés dans les ouvrages Microalgal biotechnology: potential and production, C. Posten and C. Walter, de Gruyter, 2012 et Handbook of Microalgal Culture: Applied Phycology and Biotechnology, 2ème Édition, A. Richmond and Q. Hu, Wiley-Blackwell, 2013, auxquels le lecteur pourra se référer.

D'une manière générale, l'optimisation de la production de biomasse se base sur la croissance cellulaire de la culture de microalgues. La concentration x (g/L) de microalgues dans le milieu de culture évolue en fonction du taux de croissance spécifique $\mu(x)$ ($h^{-1}$) (décrit plus loin), du taux de respiration r et du taux de dilution D du milieu de culture.

La respiration correspond à l'ensemble des réactions qui consomment de l'$O_2$ et les réserves carbonées (carbohydrates, lipides), et rejettent du $CO_2$ pour assurer la maintenance cellulaire. La respiration intervient notamment dans les mécanismes de la glycolyse, dans le cycle de Krebs, dans la voie des pentoses phosphates et dans la respiration mitochondriale.

Le taux de dilution D est donné par le débit (L/h) divisé par le volume (L) du milieu de culture. Le débit est défini par le débit de liquide entrant dans le bioréacteur, qui peut différer du débit de sortie (le volume est alors variable).

Ainsi, la croissance cellulaire x de la culture de microalgues peut être définie par la formule I suivante :

$$\dot{x} = \left( \mu(x) - r - D \right)x \qquad \text{(I),}$$

où

- $\dot{x}$ est la croissance cellulaire dans le réacteur ;
- $\mu(x)$ est le taux de croissance spécifique moyen, qui dépend de la biomasse à cause de l'auto-ombrage ;
- r est le taux de respiration ;
- D est le taux de dilution ;
- x est la concentration de microalgues dans la culture.

Pour mieux déterminer la qualité du système, il est intéressant de mesurer la production de biomasse nette P = Dx à l'équilibre. En tenant compte qu'à l'équilibre $\dot{x} = 0$, on obtient la formule II du taux de production de biomasse à l'équilibre ci-dessous :

$$P = Dx = \left( \mu(x) - r \right)x \qquad \text{(II),}$$

où

- P est le taux de production de biomasse;
- D est le taux de dilution;
- $\mu(x)$ est le taux de croissance spécifique ;
- x est la concentration de la culture cellulaire d'algues ;
- r est le taux de respiration.

Le taux de croissance spécifique $\mu(x)$ correspond à la croissance moyenne de l'ensemble des cellules considérées indépendamment les unes des autres, dans un volume L donné. Le taux de croissance net d'une cellule est donné par la différence entre le carbone fixé par la photosynthèse et le carbone perdu par respiration.

Chaque cellule doit donc être considérée individuellement en fonction de la lumière qu'elle reçoit. Il faut notamment considérer l'activité métabolique de chaque cellule. L'activité métabolique d'une cellule correspond à l'état dans lequel se trouve une cellule, c'est-à-dire au bilan entre son activité de photosynthèse et son activité de respiration. L'activité est dépendante de la localisation de la cellule dans le milieu de culture. En effet, selon la localisation de la cellule au sein du milieu de culture, la cellule va recevoir un nombre plus ou moins important de photons $\gamma$ (détaillé plus loin). Il s'ensuit qu'il existe un taux de croissance local $\mu_z$ qui est fonction de la localisation z (ou de la profondeur z) dans le bioréacteur de hauteur L. Ce taux de croissance local $\mu_z$ peut être déterminé en fonction du nombre de photons absorbés selon la formule III suivante:

$$\mu_z = \phi \frac{\gamma(z,x)}{\gamma(z,x) + k_1 + \gamma(z,x)^2/k_2} \tag{III},$$

où

- $\mu_z$ est le taux de croissance local à une profondeur z ;
- y est le nombre de photons absorbés par une cellule ;
- $k_1$ est une constante de saturation;
- $k_2$ est une constante d'inhibition ;
- $\Phi$ est une constante définissant le taux de croissance maximal.

Ces constantes $k_1$, $k_2$, et $\Phi$ peuvent être identifiées à partir de mesures de la réponse photosynthétique en fonction de la lumière. À titre d'exemple, $k_1$ = 7000 nmol.s$^{-1}$.kg$^{-1}$, $k_2$ = 284 000 nmol.s$^{-1}$.kg$^{-1}$ et $\Phi$ = 2 j$^{-1}$. Ces valeurs donnent un taux de croissance maximal de 1,5 j$^{-1}$ correspondant à une intensité de 500 nmol.m$^{-2}$.s$^{-1}$.

L'intégrale du taux de croissance local $\mu_z$ de l'ensemble des localisations z sur toute la profondeur L du bioréacteur fournit le taux de croissance spécifique $\mu(x)$.

Ainsi, le taux de croissance spécifique peut être déterminé par la formule III suivante :

$$\mu(x) = \frac{1}{L}\int_0^L \mu_z dz = \frac{\phi}{L}\int_0^L \frac{\gamma(z,x)}{\gamma(z,x) + k_1 + \gamma(z,x)^2/k_2} dz$$

(IV),

où

- $\mu(x)$ est le taux de croissance spécifique moyen;
- $\mu_z$ est le taux de croissance local en une profondeur z ;
- $\gamma$ est le nombre de photons absorbés par une cellule ;
- $k_1$ est une constante de saturation;
- $k_2$ est une constante d'inhibition ;
- x est la concentration de la culture cellulaire d'algues ;
- z est la localisation (ou profondeur) d'une cellule ;
- $\Phi$ est une constante définissant le taux de croissance maximal ;
- L est la hauteur du bioréacteur.

Le nombre de photons $\gamma$ captés par une cellule est dépendant de plusieurs paramètres.

Il faut considérer l'intensité I de la lumière reçue par une cellule. L'intensité varie selon la longueur d'onde $\lambda$, la concentration x de la culture de microalgues et la localisation z de la cellule. L'intensité I peut être qualifiée par l'atténuation de lumière due à l'absorption et à la diffusion de lumière par les autres cellules situées plus près de la source de lumière (plus en surface).

En effet, dans une culture cellulaire, une lumière incidente est émise avec une certaine intensité $I_{in}$ (dite « intensité entrante »). Le gradient de lumière au sein de la cuve du bioréacteur contenant une culture cellulaire à haute densité a pour conséquence une variation de l'intensité de lumière au sein de ladite cuve. L'intensité de la lumière en sortie $I_{out}$ (dite « intensité sortante ») est atténuée par rapport à l'intensité $I_{in}$.

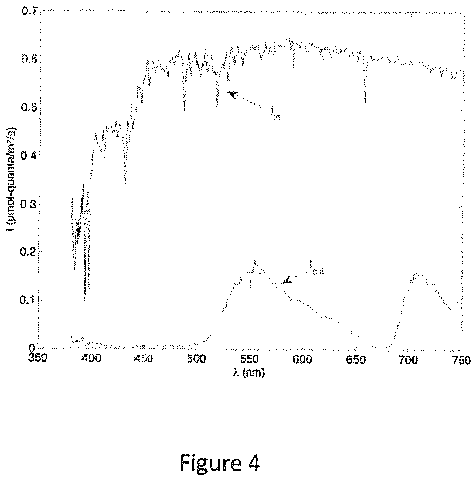

La figure 4 montre un graphique de l'intensité de la lumière incidente $I_{in}$ et l'intensité de la lumière sortante $I_{out}$ en fonction de la longueur d'onde $\lambda$, obtenue pour une culture en lumière naturelle de *Dunaliella salina.* On distingue un maximum d'intensité de la lumière sortante pour des longueurs d'ondes comprises entre 525 nm et 575 nm.

L'intensité I de la lumière captée par une cellule est donc dépendante de l'intensité de la lumière incidente $I_{in}$ de longueur d'onde $\lambda$ mais aussi du profil d'atténuation (combinaison d'absorption et de diffusion) de la culture cellulaire de microalgues, de la concentration x et de la localisation z. Par exemple, pour un système de culture plan, l'intensité de la lumière reçue localement en une profondeur z par une cellule peut être exprimée par la formule IV suivante :

$$I(\lambda, x, z) = I_{in}(\lambda)\exp(-K(\lambda)xz)$$

(V),

où

- I est l'intensité de la lumière ;
- $I_{in}$ est l'intensité de la lumière incidente ;
- $\lambda$ est la longueur d'onde ;
- K est le spectre d'atténuation ;
- x est la concentration de la culture cellulaire d'algues ;
- z est la localisation (ou profondeur) d'une cellule.

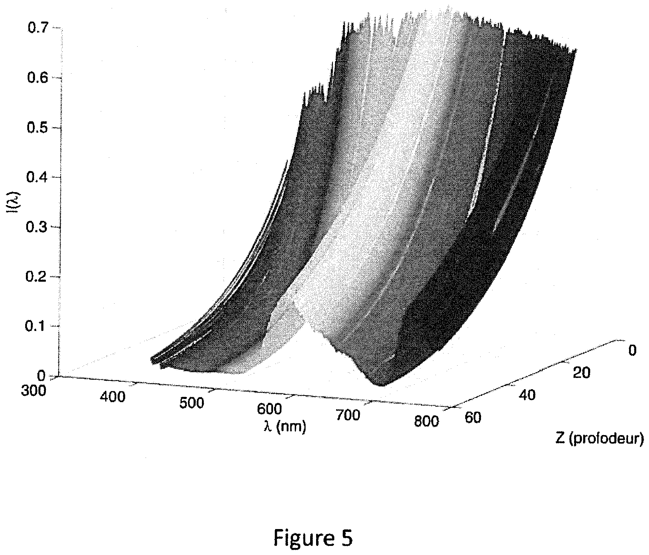

La figure 5 illustre le gradient de lumière dans un bioréacteur plan. Ainsi, la figure 5 montre l'intensité I($\lambda$) en fonction de la longueur d'onde $\lambda$ et en fonction de la profondeur z de pénétration dans un milieu de culture.
Pour connaître le nombre de photons absorbés $\gamma$ par une cellule localisée en une profondeur z dans une cuve de bioréacteur contenant une culture cellulaire de concentration x, il faut considérer sur l'ensemble des longueurs d'ondes reçues par cette cellule, l'intensité de la lumière reçue et l'absorbance pour lesdites longueurs d'ondes reçues.
Ainsi, le nombre de photons absorbés est défini par la formule V suivante :

$$\gamma(z, x) = \int_{\lambda_{min}}^{\lambda_{max}} I(\lambda, x, z)A(\lambda)d\lambda$$

(VI),

où

- $\gamma$ est le nombre de photons absorbés;
- $\lambda_{min}$ est la longueur d'onde minimale ;
- $\lambda_{max}$ est la longueur d'onde maximale ;
- A est le spectre d'absorption ;
- x est la concentration de la culture cellulaire d'algues ;
- z est la localisation (ou profondeur) d'une cellule.

En conséquence, lorsqu'on connaît l'intensité de la lumière incidente, le profil d'absorption des cellules, et leurs paramètres cinétiques, on peut évaluer le taux de production au moyen des formules I à VI ci-dessus.
À partir de ce constat, la Demanderesse a réalisé des simulations de la productivité pour des lumières incidentes de différentes longueurs d'ondes.

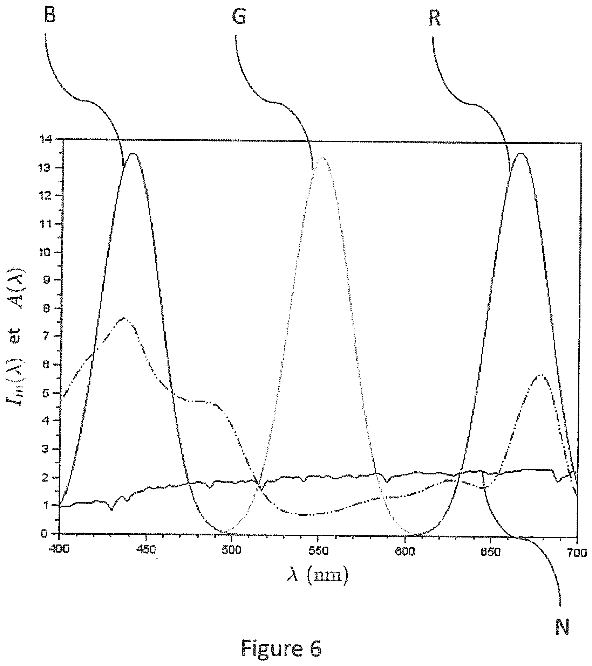

La figure 6 montre l'absorbance A($\lambda$) d'une microalgue de type algue verte ainsi que quatre spectres différents de lumières incidentes $I_{in}(\lambda)$ (ayant un nombre égal de photons) en fonction de la longueur d'onde. Les quatre lumières sont respectivement la lumière naturelle (N) (spectre AM 1.5 de *American Society for Testing and Materials* représentant le spectre solaire) ; la lumière bleue (B) ; la lumière verte (G) ; et la lumière rouge (R).
Les lumières incidentes bleue et rouge correspondent à des valeurs élevées d'absorption, alors que la lumière verte correspond à une zone de faible absorption.

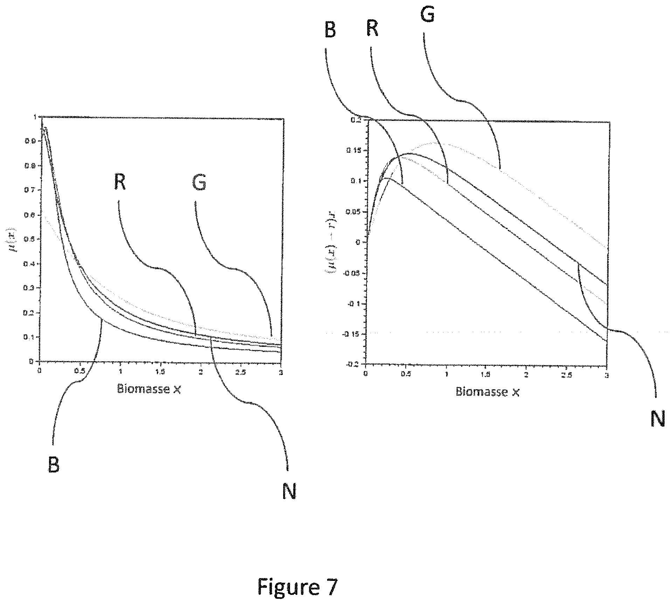

La figure 7 montre la comparaison entre les différentes lumières du taux de croissance spécifique [$\mu(x)$] de la biomasse et de la productivité nette [$(\mu(x) - r)x$] en fonction de la concentration cellulaire. À basse densité cellulaire (concentration inférieure à environ 0,5 g/l) une lumière verte G donne un taux de croissance plus faible que la lumière naturelle N, la lumière bleue B ou la lumière rouge R.
Cependant, la Demanderesse a découvert non sans surprise qu'à haute densité cellulaire (concentration supérieure à environ 0,5 g/l) la lumière verte donne un taux de croissance moyen dans la culture plus fort que la lumière naturelle N, la lumière bleue B ou la lumière rouge R.
Ceci a motivé la Demanderesse d'étudier des lumières présentant des photons de longueur d'onde bien spécifique. Des lumières favorables pour augmenter le taux de croissance moyen dans les cultures à haute densité cellulaire ont ainsi été identifiées.
Tout particulièrement, une lumière dont plus de 60% des photons ont une longueur d'onde comprise entre 500 nm et 650 nm, préférentiellement entre 520 nm et 630 nm, plus préférentiellement entre 540 nm et 610 nm, et plus préférentiellement encore entre 540 nm et 570 nm, est mise en œuvre dans la présente invention.

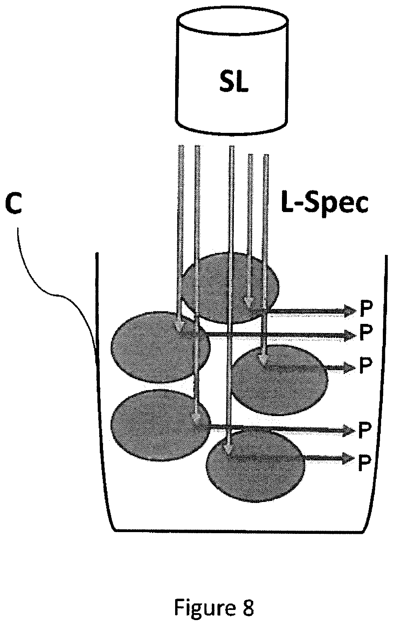

La figure 8 montre un schéma de production P de biomasse de microalgues dans un bioréacteur selon l'invention. L'utilisation d'une lumière incidente L-Spec dont plus de 60% des photons présentent une longueur d'onde comprise entre 500 nm et 650 nm, préférentiellement entre 520 nm et 630 nm, plus préférentiellement entre 540 nm et 610 nm, et plus préférentiellement encore entre 540 nm et 570 nm présente plusieurs avantages :

- L'utilisation d'une lumière de longueur d'onde comprise dans les plages citées ci-dessus permet de limiter les effets de dissipation non-photochimique NPQ et de photoinhibition.

- Ensuite, la faible absorption de la lumière par les cellules mise en œuvre selon l'invention a pour conséquence que les photons ne sont pas absorbés en totalité par les cellules en surface. Les photons peuvent donc pénétrer en profondeur du milieu de culture dans la cuve C. En d'autres termes, une grande partie des photons pénètre en profondeur et fournit de l'énergie aux cellules situées en profondeur. La photosynthèse est donc sensiblement mieux répartie sur l'ensemble des cellules de microalgues.

- L'utilisation de lumière de longueur d'onde comprise dans les plages citées ci-dessus évite des pertes d'énergie normalement liées au surplus de photons non métabolisés par les cellules d'algues.

- La faible proportion de lumière bleu et rouge entraîne une activation réduite des photorécepteurs coordonnant le cycle cellulaire, ayant pour conséquence une augmentation de la taille cellulaire et une augmentation du contenu en carbone de réserve.

- Enfin, la meilleure répartition des photons permet une culture à forte concentration cellulaire, ce qui limite les besoins énergétiques pour agiter la culture, facilite la récolte des microalgues et diminue les besoins en eau. Ainsi l'invention permet d'opérer sur des cultures cellulaires de concentration supérieure à 0,1 g/L dans des bioréacteurs ouverts. Les bioréacteurs ouverts comportent des bassins à ciel ouvert qui présentent généralement une forme de piste ovale. Ces bioréacteur ouverts sont communément connus sous le nom de bioréacteurs de type raceway. L'invention permet aussi d'opérer sur des cultures cellulaires de concentration supérieure à 0,5 g/L dans les bioréacteurs fermés. Dans un mode de réalisation particulier, la concentration de la culture cellulaire a une concentration supérieure à 1 g/L.

Le choix de la lumière spectrale L-Spec peu absorbée est fonction de l'espèce de microalgues cultivée. En effet, c'est le spectre d'absorption de l'espèce de microalgues qui détermine la sélection de la lumière spectrale. Par exemple, pour les algues vertes (contenant de la Chlorophylle a et b), la lumière verte est peu absorbée. Un spectre de lumière compris entre 530 nm et 600 nm peut être utilisé. Pour une microalgue ne contenant pas de chlorophylle b (telle que les diatomées), un spectre de lumière entre 530 nm et 650 nm peut être utilisé.

Ainsi le bioréacteur de l'invention comporte une source de lumière SL et une cuve C. La cuve comprend un milieu de culture dans lequel est dispersée une culture cellulaire constituée de cellules d'algues. La culture cellulaire d'algues présente une concentration supérieure à 0,1 g/L dans le milieu de culture et chaque cellule d'algue présente une absorption minimale dans une plage de longueurs d'onde spécifique de lumière L-Spec. La source de lumière SL est capable d'émettre une lumière incidente en direction de la cuve C. Cette lumière incidente est constituée d'au moins 60% de photons dont la longueur d'onde est comprise dans la plage de longueur d'onde spécifique de lumière L-Spec.

Chaque cellule d'algue peut notamment présenter une absorption minimale dans une plage de longueur d'onde spécifique de lumière comprise entre 500 nm et 650 nm, préférentiellement comprise entre 520 nm et 630 nm, plus préférentiellement entre 540 nm et 600 nm, et encore plus préférentiellement entre 540 nm et 570 nm. La lumière incidente du bioréacteur est alors constituée respectivement d'au moins 60% de photons dont la longueur d'onde est comprise entre 500 nm et 650 nm, préférentiellement comprise entre 520 nm et 630 nm, plus préférentiellement entre 540 nm et 600 nm, et encore plus préférentiellement entre 540 nm et 570 nm.

La source de lumière peut être constituée d'un ou plusieurs panneaux à diodes électroluminescentes (LED, en anglais : Light Emitting Diode). Chaque panneau à diodes électroluminescentes émet alors au moins 60% de photons dont la longueur d'onde est comprise entre 500 nm et 650 nm, préférentiellement comprise entre 520 nm et 630 nm, plus préférentiellement entre 540 nm et 600 nm, et encore plus préférentiellement entre 540 nm et 570 nm.

La source de lumière peut aussi être composée de lumière solaire couplée à au moins un panneau photovoltaïque semi-transparent. Chaque panneau photovoltaïque semi-transparent est alors agencé pour filtrer la lumière solaire et ne laisser passer qu'une lumière incidente constituée d'au moins 60% de photons dont la longueur d'onde est comprise entre 500 nm et 650 nm, préférentiellement comprise entre 520 nm et 630 nm, plus préférentiellement entre 540 nm et 600 nm, et encore plus préférentiellement entre 540 nm et 570 nm.

Le milieu de culture peut également comporter une source de carbone organique (culture en mixotrophie).

Le bioréacteur peut être conduit par un mode d'alimentation choisi parmi le groupe constitué d'un mode d'alimentation par batch, d'un mode d'alimentation par fed-batch et d'un mode d'alimentation continu.

Selon un mode de réalisation le bioréacteur est de type ouvert (raceway).

Selon un autre mode de réalisation, le bioréacteur est de type fermé. Dans ce mode de réalisation, la culture cellulaire d'algues présente de préférence une concentration supérieure à 0,5 g/L.

La culture cellulaire d'algues peut présenter une concentration supérieure à 1,0 g/L.

Dans un mode de réalisation, la source de lumière peut être de type LED. Ce type de source de lumière permet de générer des spectres lumineux étroits, ce qui permet de cibler une plage précise de longueurs d'onde.

Dans un autre mode de réalisation, la source de lumière peut être de la lumière solaire couplée à des panneaux photovoltaïques semi-transparents. Dans ce mode, les panneaux laissent passer, par filtration optique, la plage du spectre peu absorbée par les microalgues. Avantageusement, les plages de spectre absorbées par les panneaux sont utilisées pour la production d'électricité photovoltaïque.

Selon un mode de réalisation, le bioréacteur de l'invention peut être opéré en mode semi-continu ou continu (aussi appelé chemostat). Dans ce mode de réalisation, le bioréacteur maintient une haute densité de cellules dans le milieu de culture afin de capter la quasi-totalité de la lumière. La concentration élevée de biomasse permet par ailleurs de faciliter les opérations de récolte, par exemple par centrifugation ou filtration.

Une procédure permet d'optimiser la concentration des microalgues de la culture de manière à maximiser la productivité.

La concentration de cellules dans le milieu de culture est un paramètre clé de la production de biomasse. Une concentration trop faible a pour conséquence qu'une partie de l'énergie lumineuse n'est pas captée par les cellules. Une concentration trop élevée a pour conséquence une respiration des cellules en profondeur du milieu de culture supérieure à la fixation de carbone par photosynthèse. L'un ou l'autre cas affecte négativement la productivité de biomasse. La concentration optimale dépend de la source lumineuse, de la géométrie du réacteur et de l'espèce cultivée. Elle est généralement comprise entre 0,5 et 2 g/L.

En mode opérationnel continu et avec un apport de lumière constant dans une plage de longueur d'onde définie, le procédé de production de biomasse dans un mode de réalisation de l'invention comprend les étapes suivantes :

1. Placer un milieu de culture (par exemple de type 5F) dans un photobioréacteur (tubulaire, plan, ou colonne...) ou un bassin de culture;

2. Inoculer le milieu de culture avec une espèce de microalgues choisie (par exemple *Chlorella vulgaris, Dunaliella salina, Haematococcus pluvialis, Arthrospira platensis*) ;

3. Opérer le bioréacteur en mode batch jusqu'à l'obtention d'une biomasse importante (concentration de l'ordre de 1 g/L par exemple) ;

4. Opérer avec un taux de dilution constant $D_0$ jusqu'à atteindre un équilibre (à savoir une concentration constante de biomasse), puis mesurer la productivité obtenue ;

5. Fixer un facteur de variation du taux de dilution p%, de préférence 30% ;

6. Opérer avec un nouveau taux de dilution p% supérieur (respectivement p% inférieur) jusqu'à atteindre un équilibre ;

7. Mesurer la productivité à cet équilibre et :

    a. si les sens de variation du taux de dilution et de la productivité sont opposés, réduire de moitié le facteur de variation du taux de dilution p%.

    b. si la productivité a augmenté, recommencer l'étape 6 en augmentant (respectivement en diminuant) à nouveau le taux de dilution ;

    c. si la productivité a diminué, recommencer l'étape 6 en diminuant (respectivement en augmentant) le taux de dilution.

Généralement, le facteur de variation du taux de dilution doit être réinitialisé de manière périodique ou en cas de perturbation.

Le bioréacteur peut comporter un système de pilotage du taux de dilution (une pompe dont le débit est ajustable de manière manuelle ou commandée).

Les variations du taux de dilution peuvent être adaptées en fonction des écarts de productivité. Si l'écart est important, le système est loin du point optimal de fonctionnement donc le taux de dilution peut être modifié de manière significative. À l'inverse, si l'écart de productivité entre deux taux de dilution est faible, le système est proche de l'optimum et de faibles variations du taux de dilution peuvent être réalisées. Le taux de dilution sera de préférence automatiquement adapté de sorte que la perte de carbone par respiration dans les zones sombres du réacteur compense sensiblement la fixation de carbone par photosynthèse.

Les mêmes étapes peuvent être réalisées dans un mode opérationnel dit turbidostat : le taux de dilution est ajusté de manière à réguler à une valeur de référence la concentration de biomasse (de l'ordre de 0,5 à 2 g/L) ou l'atténuation lumineuse (entre 90 et 99%). Le choix de la consigne (atténuation lumineuse ou concentration de biomasse) est adapté afin d'optimiser la productivité, en suivant la même procédure d'ajustement.

Dans les modes opérationnels de type lumière jour/nuit, les étapes ci-dessus peuvent être employées avec les modifications suivantes :

- Le taux de dilution (ou la consigne, en mode turbidostat) est appliqué jusqu'à atteindre un régime périodique, à savoir la même concentration cellulaire d'un jour à l'autre à la même heure ;
- La productivité est calculée sur une période, en intégrant la productivité instantanée sur une période.

L'évaluation de la productivité est réalisée par un ou plusieurs capteurs du bioréacteur, ou manuellement.

L'évaluation peut être réalisée en sortie du réacteur (par exemple par mesures de débits et de biomasses par poids secs, par mesures de densité optique, par comptage cellulaire, etc.). L'évaluation de la productivité peut aussi être réalisée par des capteurs disposés dans le bioréacteur pour mesurer la phase liquide (capteur d'oxygène, capteur de dioxyde de carbone, etc.) ou la phase gazeuse en sortie du réacteur.

Un choix pertinent consiste à réguler le pH dans le milieu de culture par un apport contrôlé en $CO_2$ à partir d'une mesure de pH. La régulation du pH assure que la quantité de $CO_2$ dissous sera constante. Il est alors possible d'évaluer la productivité du système par la quantité totale de $CO_2$ injectée sur une certaine période.

Le bioréacteur de l'invention peut donc être opéré en mode continu. Dans ce mode de réalisation le taux de dilution est ajusté manuellement ou automatiquement de manière à maximiser la productivité estimée par la production d'oxygène, la consommation de $CO_2$ ou par mesure de la biomasse in situ (capteur de turbidité) ou après prélèvements (densité optique, poids sec, etc.).

Le procédé décrit ci-dessus peut être mis en œuvre dans un bioréacteur selon l'invention. L'invention vise donc aussi une utilisation du bioréacteur tel que défini ci-dessus pour la production de biomasse d'une culture cellulaire d'algues. La culture cellulaire d'algues est choisie de préférence parmi le groupe constitué des classes Chlorophyceae telles que *Chlamydomonas sp.*, *Dunaliella salina* et *Haematococcus pluvialis*, Bacillariophyceae (Diatomophyceae) telles que *Phaeodactylum tricornutum* et *Odontella aurita*, Isochrysidaceae telles que *Isochrysis galbana* et *Tisochrysis lutea*, Trebouxiophyceae telle que *Chlorella vulgaris*, et Cyanophyceae telle que *Arthrospira platensis.*

## Revendications

1. Bioréacteur comportant une source de lumière (SL) et une cuve (C) comprenant un milieu de culture dans laquelle est dispersée une culture cellulaire constituée de cellules d'algues, ladite culture cellulaire d'algues présentant une concentration supérieure à 0,1 g/L dans le milieu de culture, chaque cellule d'algue présentant une absorption minimale dans une plage de longueurs d'onde spécifique de lumière (L-Spec) comprise entre 500 nm et 650 nm, ladite source (SL) étant capable d'émettre une lumière incidente en direction de la cuve (C) constituée d'au moins 60% de photons dont la longueur d'onde est comprise dans la plage de longueur d'onde spécifique de lumière (L-Spec).

2. Bioréacteur selon la revendication 1, dans lequel chaque cellule d'algue présente une absorption minimale dans une plage de longueur d'onde spécifique de lumière comprise entre 520 nm et 630 nm, et plus préférentiellement entre 540 nm et 570 nm.

3. Bioréacteur selon l'une des revendications précédentes, dans lequel la lumière incidente est constitué d'au moins 60% de photons dont la longueur d'onde est comprise entre 500 nm et 650 nm, préférentiellement comprise entre 520 nm et 630 nm, et plus préférentiellement entre 540 nm et 570 nm.

4. Bioréacteur selon l'une des revendications 1 à 3, dans lequel la source de lumière est constituée d'au moins un panneau à diodes électroluminescentes, chaque panneau à diodes électroluminescentes émettant au moins 60% de photons dont la longueur d'onde est comprise entre 500 nm et 650 nm, préférentiellement comprise entre 520 nm et 630 nm, et plus préférentiellement entre 540 nm et 570 nm.

5. Bioréacteur selon l'une des revendications 1 à 3, dans lequel la source de lumière est composée de lumière solaire couplée à au moins un panneau photovoltaïque semi-transparent, chaque panneau photovoltaïque semi-transparent étant apte à filtrer la lumière solaire et ne laisser passer qu'une lumière incidente constitué d'au moins 60% de photons dont la longueur d'onde est comprise entre 500 nm et 650 nm, préférentiellement comprise entre 520 nm et 630 nm, et plus préférentiellement entre 540 nm et 570 nm.

6. Bioréacteur selon l'une des revendications précédentes, dans lequel le milieu de culture comporte une source de carbone organique.

7. Bioréacteur selon l'une des revendications précédentes, dans lequel le bioréacteur est conduit par un mode d'alimentation choisi parmi le groupe constitué d'un mode d'alimentation par batch, d'un mode d'alimentation par fed-batch et d'un mode d'alimentation continu.

8. Bioréacteur selon l'une des revendications précédentes, le bioréacteur étant de type ouvert.

9. Bioréacteur selon l'une des revendications 1 à 7, le bioréacteur étant de type fermé et la culture cellulaire d'algues présentant une concentration supérieure à 0,5 g/L.

10. Bioréacteur selon l'une des revendications précédentes, dans lequel la culture cellulaire d'algues présente une concentration supérieure à 1,0 g/L.

11. Utilisation d'un bioréacteur selon l'une des revendications 1 à 10 pour la production de biomasse d'une culture cellulaire d'algues.

12. Utilisation selon la revendication 11, dans laquelle la culture cellulaire d'algues est choisie parmi le groupe constitué des classes Chlorophyceae telles que *Chlamydomonas sp.*, *Dunaliella salina* et *Haematococcus pluvialis*, Bacillariophyceae (Diatomophyceae) telles que *Phaeodactylum tricornutum* et *Odontella aurita*, Isochrysidaceae telles que *Isochrysis galbana* et *Tisochrysis lutea*, Trebouxiophyceae telle que *Chlorella vulgaris*, et Cyanophyceae telle que *Arthrospira platensis.*

**Patentansprüche**

1. Bioreaktor, umfassend eine Lichtquelle (SL) und einen ein Kulturmedium umfassenden Tank (C), in dem eine Zellkultur dispergiert ist, die aus Algenzellen besteht, wobei die Algenzellkultur eine Konzentration von mehr als 0,1 g/L in dem Kulturmedium aufweist, wobei jede Algenzelle eine minimale Absorption in einem spezifischen Lichtwellenlängenbereich (L-Spec) aufweist, der zwischen 500 nm und 650 nm liegt, wobei die Quelle (SL) in der Lage ist, ein einfallendes Licht in der Richtung des Tanks (C) abzustrahlen, das zu mindestens 60 % aus Photonen besteht, deren Wellenlänge in dem spezifischen Lichtwellenlängenbereich (L-Spec) liegt.

2. Bioreaktor nach Anspruch 1, wobei jede Algenzelle eine minimale Absorption in einem spezifischen Lichtwellenlängenbereich aufweist, der zwischen 520 nm und 630 nm und mehr bevorzugt zwischen 540 nm und 570 nm liegt.

3. Bioreaktor nach einem der vorhergehenden Ansprüche, wobei das einfallende Licht zu mindestens 60 % aus Photonen besteht, deren Wellenlänge zwischen 500 nm und 650 nm liegt, bevorzugt zwischen 520 nm und 630 nm und mehr bevorzugt zwischen 540 nm und 570 nm liegt.

4. Bioreaktor nach einem der Ansprüche 1 bis 3, wobei die Lichtquelle aus mindestens einem Lumineszenzdiodenpanel besteht, wobei jedes Lumineszenzdiodenpanel zu mindestens 60 % Photonen abstrahlt, deren Wellenlänge zwischen 500 nm und 650 nm liegt, bevorzugt zwischen 520 nm und 630 nm und mehr bevorzugt zwischen 540 nm und 570 nm liegt.

5. Bioreaktor nach einem der Ansprüche 1 bis 3, wobei die Lichtquelle sich aus Sonnenlicht zusammensetzt, das an mindestens ein teildurchlässiges Photovoltaikpanel gekoppelt ist, wobei jedes teildurchlässige Photovoltaikpanel in der Lage ist, das Sonnenlicht zu filtern und nur ein einfallendes Licht passieren zu lassen, das zu mindestens 60 % aus Photonen besteht, deren Wellenlänge zwischen 500 nm und 650 nm liegt, bevorzugt zwischen 520 nm und 630 nm und mehr bevorzugt zwischen 540 nm und 570 nm liegt.

6. Bioreaktor nach einem der vorhergehenden Ansprüche, wobei das Kulturmedium eine organische Kohlenstoffquelle umfasst.

7. Bioreaktor nach einem der vorhergehenden Ansprüche, wobei der Bioreaktor durch einen Zufuhrmodus gesteuert wird, der aus der Gruppe bestehend aus einem Batch-Zufuhrmodus, einem Fed-Batch-Zufuhrmodus und einem kontinuierlichen Zufuhrmodus ausgewählt ist.

8. Bioreaktor nach einem der vorhergehenden Ansprüche, wobei der Bioreaktor vom offenen Typ ist.

9. Bioreaktor nach einem der Ansprüche 1 bis 7, wobei der Bioreaktor vom geschlossenen Typ ist und die Algenzellkultur eine Konzentration von mehr als 0,5 g/L aufweist.

10. Bioreaktor nach einem der vorhergehenden Ansprüche, wobei die Algenzellkultur eine Konzentration von mehr als 1,0 g/L aufweist.

11. Verwendung eines Bioreaktors nach einem der Ansprüche 1 bis 10 zur Produktion einer Biomasse aus einer Algenzellkultur.

12. Verwendung nach Anspruch 11, wobei die Algenzellkultur aus der Gruppe bestehend aus Klassen von Chlorophyceae, wie *Chlamydomonas sp.*, *Dunaliella salina* und *Haematococcus pluvialis,* Bacillariophyceae (Diatomophyceae), wie *Phaedactylum tricornutum* und *Odontella aurita,* Isochrysidaceae, wie *Isochrysis galbana* und *Tisochrysis lutea*, Trebouxiophyceae, wie *Chlorella vulgaris*, und Cyanophyceae, wie *Arthrospira platensis*, ausgewählt ist.

## Claims

1. A bioreactor comprising a light source (LS) and a tank (T) comprising a culture medium in which a cell culture consisting of algal cells is dispersed, said algal cell culture having a concentration of greater than 0.1 g/l in the culture medium, each algal cell having a minimum absorption in a light-specific range of wavelengths (L-Spec) of between 500 nm and 650 nm, said source (LS) being capable of emitting incident light in the direction of the tank (T) consisting of at least 60% of photons having a wavelength within the light-specific range of wavelengths (L-Spec).

2. The bioreactor as claimed in claim 1, wherein each algal cell has a minimum absorption in a light-specific range of wavelengths of between 520 nm and 630 nm, and more preferentially between 540 nm and 570 nm.

3. The bioreactor as claimed in one of the preceding claims, wherein the incident light consists of at least 60% of photons having a wavelength of between 500 nm and 650 nm, preferentially between 520 nm and 630 nm, and more preferentially between 540 nm and 570 nm.

4. The bioreactor as claimed in one of claims 1 to 3, wherein the light source consists of at least one light-emitting diode panel, each light-emitting diode panel emitting at least 60% of photons having a wavelength of between 500 nm and 650 nm, preferentially between 520 nm and 630 nm, and more preferentially between 540 nm and 570 nm.

5. The bioreactor as claimed in one of claims 1 to 3, wherein the light source is composed of sunlight coupled to at least one semi-transparent photovoltaic panel, each semi-transparent photovoltaic panel being able to filter sunlight and only allow passage of incident light consisting of at least 60% of photons having a wavelength of between 500 nm and 650 nm, preferentially between 520 nm and 630 nm, and more preferentially between 540 nm and 570 nm.

6. The bioreactor as claimed in one of the preceding claims, wherein the culture medium comprises an organic carbon source.

7. The bioreactor as claimed in one of the preceding claims, wherein the bioreactor is driven by a supply mode selected from the group consisting of a batch supply mode, a fed-batch supply mode and a continuous supply mode.

8. The bioreactor as claimed in one of the preceding claims, the bioreactor being of the open type.

9. The bioreactor as claimed in one of claims 1 to 7, the bioreactor being of the closed type and the algal cell culture having a concentration of greater than 0.5 g/l.

10. The bioreactor as claimed in one of the preceding claims, wherein the algal cell culture has a concentration of greater than 1.0 g/l.

11. The use of a bioreactor as claimed in one of claims 1 to 10 for the production of biomass from an algal cell culture.

12. The use as claimed in claim 11, wherein the algal cell culture is selected from the group consisting of the Chlorophyceae classes, such as *Chlamydomonas sp.*, *Dunaliella salina* and *Haematococcus pluvialis*, Bacillariophyceae (Diatomophyceae) such as *Phaeodactylum tricornutum* and *Odontella aurita*, Isochrysidaceae such as *Isochrysis galbana* and *Tisochrysis lutea*, Trebouxiophyceae such as *Chlorella vulgaris*, and Cyanophyceae such as *Arthrospira platensis.*

Figure 1

Figure 2

Figure 3

Figure 4

Figure 5

Figure 6

Figure 7

Figure 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010085853 A

**Littérature non-brevet citée dans la description**

- **CARVALHO et al.** Light requirements in microalgal photobioreactors: an overview of biophotonic aspects. *Appl Microbiology and Biotechnology,* 2011, vol. 89 (5), 1275-1288
- **SCHULZE et al.** Light emitting diodes (LEDs) applied to microalgal production. *Trends in Biotechnology,* 2014, vol. 32 (8), 422-430
- **MÜLLER et al.** Non-Photochemical Quenching. A Response to Excess Light Energy. *Plant Physiol,* 2001, vol. 125 (4), 1558-1566
- **STOMP et al.** Colorful niches of phototrophic microorganisms shaped by vibrations of the water molecule. *The ISME journal,* 2007, vol. 1 (4), 271-282
- **RUDIGER, W ; LOPEZ-FIGUEROA, F.** Photoreceptors in algae. *Photochemistry and photobiology,* 1992, vol. 55, 949-954
- **TAMBURIC et al.** Action spectra of oxygen production and chlorophyll a fluorescence in the green microalga Nannochloropsis oculata. *Bioresource Technology,* 2014, vol. 169, 320-327
- **FALKOWSKI, P. G. ; RAVEN, J. A.** Aquatic photosynthesis. Princeton University Press, 2013
- Microalgal biotechnology: potential and production. **C. POSTEN ; C. WALTER ; DE GRUYTER ; A. RICHMOND ; Q. HU.** Handbook of Microalgal Culture: Applied Phycology and Biotechnology. Wiley-Blackwell, 2012